# FASCICULE DE BREVET EUROPEEN

(11) **EP 1 002 530 B1**
(45) Date de publication et mention de la délivrance du brevet: **15.04.2009**
(21) Numéro de dépôt: 99402023.8
(22) Date de dépôt: 09.08.1999
(51) Int. Cl.: A61K 9/70, A61K 8/368, A61K 8/37, A61K 31/60, A61Q 19/00

(54) **Système de dèlivrance d'actif comprenant, sur une structure en film, une composition à base d'un dérivé d'acide salicylique, et ses utilisations.**
Abgabesysteme für aktive Bestandteile die eine Salicylsäurederivat-Zusammensetzung auf einem Film enthalten und ihre Verwendung
Delivery systems for active ingredients comprising a salicylic acid derivative composition on a film structure and their use

(30) Priorité: 23.09.1998 FR 9811883
(43) Date de publication de la demande: 24.05.2000
(73) Titulaire: L'ORÉAL, 75008 Paris (FR)
(72) Inventeur: Laugier, Jean-Pierre, 92160 Antony (FR); Ayache, Liliane, 75012 Paris (FR)
(74) Mandataire: Le Coupanec, Pascale A.M.P.

(56) Documents cités:
- EP-A- 0 870 498
- EP-A- 0 933 077
- WO-A-96/23490
- WO-A-97/48387
- FR-A- 2 581 542
- FR-A- 2 732 595
- FR-A- 2 755 011
- DATABASE WPI Week 9325 Derwent Publications Ltd., London, GB; AN 93-200437 XP002106578 "Keratolytic hard ointment with reduced cure time - contains salicylic acid, lanolin, fatty acid alkyl ester and adhesive tape" & JP 05 124959 A (TAISHO PHARM), 21 mai 1993 (1993-05-21)

## Description

La présente invention concerne un système de délivrance d'un actif sur la peau comprenant, sur une structure en film, une composition à base d'un dérivé d'acide salicylique ne renfermant pas d'actif hydrosoluble sous forme de particules, ledit système de délivrance assurant, par application sur la peau, la libération du dérivé d'acide salicylique dans l'épiderme. L'invention se rapporte également à l'utilisation du système précité dans le traitement cosmétique de la peau, et en particulier dans le traitement de l'acné.

On recherche en cosmétique et en dermatologie des composés ayant une activité kératolytique et/ou comédolytique, notamment dans le traitement des troubles de la kératinisation, tels que le psoriasis, l'ichtyose, l'eczéma, les kératoses actiniques et les dermatites séborrhéiques, et dans le traitement des verrues et des ulcères. Si l'utilisation de produits kératolytiques, tels que l'acide salicylique, présente également un intérêt dans le traitement de l'acné, on recherche des moyens permettant simultanément d'avoir une action anti-bactérienne et/ou hormonale, susceptibles d'agir sur les germes responsables de l'inflammation du follicule pilo-sébacé et/ou sur les hormones endogènes, essentiellement les androgènes, responsables de la production excessive de sébum.

Il est ainsi décrit dans WO-A-97/48387 un dispositif sous la forme d'un patch pour l'application topique d'une formulation anti-acné comprenant des quantités efficaces d'au moins deux ingrédients actifs choisis dans au moins deux groupes différents d'actifs, lesdits groupes étant choisis parmi les kératolytiques, tels que l'acide salicylique, les anti-irritants, les antiseptiques, les anti-microbiens, les hormones et les agonistes et antagonistes d'hormones.

Toutefois, ce type de patch n'agit pas assez rapidement sur les petits boutons. Précisément, son effet asséchant et son activité anti-inflammatoire en vingt-quatre heures ne sont pas suffisants. Des résultats satisfaisants ne peuvent être obtenus qu'en combinant au moins trois principes actifs agissant sur toutes les composantes de l'acné, à savoir un kératolytique, un antiseptique et un anti-inflammatoire. Or, l'association de plusieurs actifs de ce type augmente le risque d'intolérance du produit, ainsi que son coût de fabrication.

On connaît de FR-A-2 581 542 des dérivés d'acide salicylique substitués par un groupement acyle, lesquels ont une activité kératolytique supérieure à l'acide salicylique, à des concentrations moindres, ainsi qu'une activité bactériostatique spécifique et efficace vis-à-vis de certains germes de l'acné. Ces dérivés d'acide salicylique peuvent être mis en oeuvre dans une composition cosmétique ou dermatologique destinée à une application topique.

Dans la demande FR-A-2 732 595, il est suggéré d'ajouter aux compositions cosmétiques contenant ces dérivés d'acide salicylique un polymère filmogène tel qu'une gomme de silicone ou un polymère fluoré, en vue de réduire l'irritation que ces composés sont susceptibles d'induire sur la peau.

Toutefois, il n'a pas été envisagé d'incorporer ces dérivés d'acide salicylique dans un dispositif tel qu'un masque ou un patch destiné à être appliqué sur la peau pendant une période de temps prolongée, par exemple pendant toute une nuit.

Il a maintenant été découvert que les dérivés acylés d'acide salicylique mentionnés précédemment avaient une efficacité supérieure, et que leur administration et leur posologie pouvaient être mieux maîtrisées, lorsqu'ils étaient appliqués sous forme de masque ou de patch ne comprenant pas de particules d'actif hydrosoluble, plutôt que sous forme de crème ou de lotion, par exemple. Il a en outre été découvert que ces dérivés d'acide salicylique permettaient de surmonter les inconvénients des patchs anti-acné de l'art antérieur, en ce sens qu'ils combinaient en un seul actif des propriétés kératolytique, antiseptique et anti-inflammatoire, réduisant ainsi la durée du traitement et augmentant son efficacité, tout en étant très bien tolérés.

La présente invention a donc pour objet un système de délivrance d'un actif sur la peau comprenant, sur une structure en film, une composition à base d'un dérivé d'acide salicylique répondant à la formule (I) suivante : 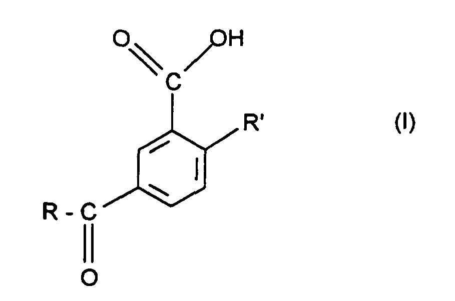 dans laquelle
R représente une chaîne aliphatique saturée ayant de 3 à 11 atomes de carbone, linéaire, ramifiée ou cyclisée, ou une chaîne insaturée ayant de 3 à 17 atomes de carbone portant une ou plusieurs doubles liaisons conjuguées ou non, ces différents substituants pouvant être substitués par un ou plusieurs atomes d'halogène, par un ou plusieurs groupements trifluorométhyle sous forme libre ou estérifiée par un acide ayant de 1 à 6 atomes de carbone ou bien par une fonction carboxyle, libre ou estérifiée par un alcool inférieur ayant de 1 à 6 atomes de carbone, et
R' représente une fonction hydroxyle ou une fonction ester comportant une chaîne saturée ou insaturée ayant de 2 à 16 atomes de carbone,
ladite composition ne renfermant pas d'actif hydrosoluble sous forme de particules, **caractérisé en ce que** ladite structure en film est constituée par, ou comprend, un matériau fibreux tissé ou non tissé, naturel ou synthétique ; ou une feuille de matière plastique sur laquelle est fixée une couche de polymère contenant ledit dérivé d'acide salicylique.

Selon une forme d'exécution préférée, R' est une fonction hydroxyle et R représente une chaîne aliphatique saturée ayant de 3 à 11 atomes de carbone. Avantageusement, le dérivé d'acide salicylique est choisi dans le groupe constitué par : l'acide n-hexanoyl-5-salicylique, l'acide n-octanoyl-5-salicylique, l'acide n-décanoyl-5-salicylique et l'acide n-dodécanoyl-5-salicylique.

Ces composés peuvent être préparés comme décrit dans FR-A-2 581 542.

D'une manière générale, le système de délivrance d'actif selon l'invention peut être utilisé à des fins cosmétiques ou pour traiter toutes les maladies de peau vis-à-vis desquelles les dérivés d'acide salicylique définis plus haut ont une activité en tant qu'agents kératolytiques et/ou antiseptiques et/ou anti-inflammatoires.

Ainsi, le système de délivrance d'actif selon l'invention peut être utilisé pour la fabrication d'un produit destiné au traitement des troubles de kératinisation tels que le psoriasis, l'ichtyose, l'eczéma, les kératoses actiniques et les dermatites séborrhéiques, ainsi qu'au traitement de l'acné, des verrues, des ulcères ou des hyperpigmentations de la peau, et plus particulièrement pour fabriquer un produit destiné au traitement de l'acné.

En variante, à une concentration plus faible d'actif, ce système de délivrance d'actif peut être utilisé à des fins de dépigmentation de la peau sans desquamation, comme décrit dans le brevet EP 747 043. Il permet ainsi de traiter avantageusement les hyperpigmentations régionales par hyperactivité mélanocytaire telles que les mélasmas idiopathiques, survenant lors de la grossesse ("masque de grossesse" ou chloasma) ou d'une contraception oestroprogestative, les hyperpigmentations localisées par hyperactivité et prolifération mélanocytaire bénigne telles que les taches pigmentaires séniles dites lentigo actiniques, les hyperpigmentations accidentelles telles que la photosensibilisation et la cicatrisation post-lésionnelle, ainsi que certaines leucodermies telles que le vitiligo.

Le système de délivrance d'actif selon l'invention peut également être utilisé pour la fabrication d'un produit destiné dans le traitement des peaux grasses et/ou des peaux jeunes à problèmes et/ou des points noirs et/ou pour assécher et/ou purifier la peau.

Le système de délivrance d'actif selon l'invention comprend une structure en film qui peut être constituée par, ou comprendre, un matériau fibreux tissé ou non tissé, naturel ou synthétique ; ou une feuille de matière plastique, constituée par exemple de polyéthylène haute ou basse densité, de polypropylène, de poly(chlorure de vinyle), de poly(téréphtalate d'éthylène), d'éthylène-acétate de vinyle ou de polyuréthane. La structure en film peut soit être respirante, soit être généralement occlusive, c'est-à-dire constituée d'une matière imperméable au dérivé d'acide salicylique, de façon à empêcher l'évaporation de ce dernier et à faciliter la pénétration de l'actif dans l'épiderme.

Ainsi, dans une forme d'exécution préférée, le système de délivrance d'actif selon l'invention peut être un patch de structure classique comportant plusieurs couches successives dans l'ordre suivant : une première couche, dite couche support, formée de la structure en film selon l'invention et qui est constituée d'une feuille plastique ; une seconde couche, dite couche de polymère, fixée sur la couche support et contenant le dérivé d'acide salicylique, cette couche pouvant venir directement au contact de la peau ; éventuellement, une troisième couche constituée d'une matière adhésive, se trouvant sur surface de la couche de polymère et perméable au composé actif ; enfin, éventuellement, une couche pelable de protection, recouvrant de façon hermétique la couche de polymère ou la couche adhésive, de façon à la protéger de toute contamination extérieure pendant le temps de stockage préalable à l'utilisation du patch.

La couche support a non seulement pour fonction de supporter la couche de polymère, mais également de servir de revêtement protecteur de celle-ci. Elle peut être de même dimension que la couche de polymère ou s'étendre au-delà de la couche de polymère et vers l'extérieur, de manière à pouvoir éventuellement recevoir des moyens adhésifs disposés à la périphérie de la couche de polymère.

Selon une première variante, la couche de polymère peut être constituée par, ou comprendre, un polymère adhésif, de sorte que la troisième couche est superflue. Il peut s'agir par exemple d'un poly(acide acrylique) tel qu'un copolymère acrylique/acétate de vinyle pouvant être obtenu auprès de NATIONAL STARCH sous la dénomination commerciale DURO-TAK^{®}. On obtient alors des patchs dits de type matriciel tels que ceux divulgués dans US-A-5 232 707, WO 96/14822 et WO 97/48387.

Dans une seconde variante, le dérivé d'acide salicylique peut être solubilisé, au sein de la couche de polymère, dans un solvant et un gélifiant. Le système de délivrance d'actif selon l'invention comprend alors, comme troisième couche, une couche adhésive perméable audit dérivé d'acide salicylique. Une membrane régulatrice peut être interposée entre ladite couche de polymère et ladite couche adhésive. On obtient ainsi un patch du type à réservoir tel que celui décrit, par exemple, dans FR-A-2 738 744.

Lorsque la couche de polymère ou la couche adhésive est protégée par une couche pelable de protection, celle-ci est enlevée au moment de l'utilisation. Elle peut être constituée de tout matériau imperméable au composé actif ainsi qu'à tout autre composant présent dans la matrice polymérique. Parmi les matériaux pouvant être utilisés, on peut citer de préférence une feuille de papier siliconée ou une feuille de matériau thermoplastique traitée pour la rendre anti-adhérente, par exemple à l'aide d'un vernis. De préférence, cette couche pelable de protection est constituée de polyéthylène.

Le système de délivrance d'actif selon l'invention peut avoir toute forme et toute dimension voulues, correspondant à l'utilisation envisagée. Ainsi, il peut avoir des dimensions réduites destinées à une application sur un bouton ou un point noir isolé ou sur une tache pigmentaire, ou des dimensions plus grandes pour une application sous forme de masque sur le visage.

Une composition à base de dérivé d'acide salicylique, répondant à la formule générale (I) donnée ci-dessus, se trouve sur la structure en film du système de délivrance d'actif selon l'invention. Cette composition renferme une quantité efficace de dérivé d'acide salicylique pour obtenir l'effet kératolytique et/ou anti-inflammatoire et/ou antiseptique souhaité. Pour une application dans le traitement de l'acné, la composition peut comprendre, par exemple, d'environ 0,01 à environ 2% en poids, de préférence d'environ 0,1 à environ 1% en poids et, mieux, environ 0,5% en poids de dérivé d'acide salicylique par rapport au poids total de la composition. Le système de délivrance d'actif selon l'invention peut ainsi délivrer d'environ 0,01 à environ 0,5 mg de dérivé d'acide salicylique par cm² de peau à traiter et être utilisé à raison de 1 à 2 applications par jour.

Comme indiqué précédemment, ces dérivés d'acide salicylique sont suffisamment efficaces en eux-mêmes pour être utilisés en tant que seul actif dans le système de délivrance d'actif selon l'invention. Toutefois, si on le souhaite, on pourra bien entendu leur ajouter d'autres actifs, tels que d'autres agents kératolytiques et/ou antiseptiques (par exemple de l'octopirox, du triclosan, etc.) et/ou anti-inflammatoires et/ou des agents anti-irritants (tels que l'acide β-glycyrrhétinique, l'a-bisabolol, etc.) et/ou anti-séborrhéiques et/ou cicatrisants, sans que cette liste soit limitative.

De façon connue, on pourra en outre ajouter au dérivé d'acide salicylique des adjuvants habituels dans le domaine cosmétique ou dermatologique, tels que les émulsionnants, les gélifiants hydrophiles ou lipophiles, les actifs hydrophiles ou lipophiles, les conservateurs, les antioxydants, les parfums, les charges, les filtres et les matières colorantes. Les quantités de ces différents adjuvants sont celles classiquement utilisées dans les domaines cosmétique et/ou dermatologique, et par exemple de 0,01 % à 20 % du poids total de la composition renfermant le dérivé d'acide salicylique.

Bien entendu, il sera évident pour l'homme du métier que les composés, actifs ou non actifs, éventuellement ajoutés aux dérivés d'acide salicylique seront choisis de manière à ne pas contrevenir au but poursuivi selon la présente invention.

Des exemples non limitatifs de fabrication de systèmes de délivrance d'actif selon l'invention sont donnés ci-après.

### Exemple 1

Un patch de type matriciel est fabriqué en ajoutant 0,5 g d'acide n-octanoyl-5-saliclique et 2 g de monooléate de sorbitane à 97,5 g de copolymère acrylique / acétate de vinyle en solution à 50% dans de l'acétate d'éthyle.

Le mélange est agité à température ambiante jusqu'à dissoudre tous les ingrédients. Après élimination des bulles d'air, le mélange est appliqué au moyen d'un dispositif d'enduction classique sur un film de polyester siliconé, puis chauffé pendant environ 15 mn au four à 75-80°C pour évaporer l'acétate d'éthyle. Une couche pelable de protection en polystyrène est ensuite appliquée sur la couche adhésive contenant l'acide n-octanoyl-5-salicylique, puis le stratifié ainsi obtenu est découpé en un patch en forme de disque, ayant une surface d'environ 1 cm².

Ce patch est efficace en application nocturne sur un bouton ou un point noir isolé.

### Exemple 2

Un patch matriciel est fabriqué de la même manière que dans l'Exemple 1, excepté que l'acide n-octanoyl-5-salicylique est remplacé par 1 g d'acide n-décanoyl-5-salicylique, la quantité de copolymère acrylique / acétate de vinyle étant réduite à 97 g.

Ce patch est efficace dans le traitement des verrues ou du psoriasis.

## Revendications

1. Système de délivrance d'un actif sur la peau comprenant, sur une structure en film, une composition à base d'un dérivé d'acide salicylique répondant à la formule (I) suivante : 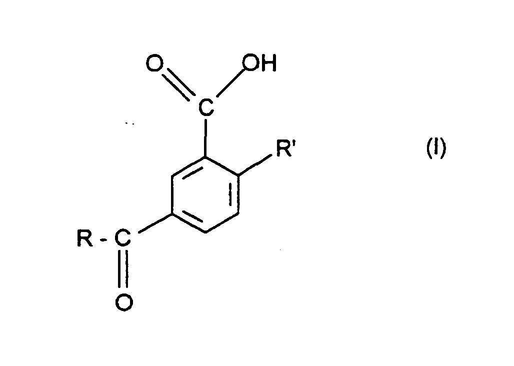 dans laquelle
R représente une chaîne aliphatique saturée ayant de 3 à 11 atomes de carbone, linéaire, ramifiée ou cyclisée, ou une chaîne insaturée ayant de 3 à 17 atomes de carbone portant une ou plusieurs doubles liaisons conjuguées ou non, ces différents substituants pouvant être substitués par un ou plusieurs atomes d'halogène, par un ou plusieurs groupements trifluorométhyle sous forme libre ou estérifiée par un acide ayant de 1 à 6 atomes de carbone ou bien par une fonction carboxyle, libre ou estérifiée par un alcool inférieur ayant de 1 à 6 atomes de carbone, et
R' représente une fonction hydroxyle ou une fonction ester comportant une chaîne saturée ou insaturée ayant de 2 à 16 atomes de carbone,
ladite composition ne renfermant pas d'actif hydrosoluble sous forme de particules, **caractérisé en ce que** ladite structure en film est constituée par, ou comprend, un matériau fibreux tissé ou non tissé, naturel ou synthétique ; ou une feuille de matière plastique sur laquelle est fixée une couche de polymère contenant ledit dérivé d'acide salicylique.

2. Système de délivrance d'actif selon la revendication 1, **caractérisé en ce que** R' est une fonction hydroxyle et R représente une chaîne aliphatique saturée ayant de 3 à 11 atomes de carbone.

3. Système de délivrance d'actif selon la revendication 2, **caractérisé en ce que** ledit dérivé d'acide salicylique est choisi dans le groupe constitué par : l'acide n-hexanoyl-5-salicylique, l'acide n-octanoyl-5-salicylique, l'acide n-décanoyl-5-salicylique et l'acide n-dodécanoyl-5-salicylique.

4. Système de délivrance d'actif selon l'une quelconque des revendications 1 à 3, **caractérisé en ce que** ladite couche de polymère est constituée d'un polymère adhésif.

5. Système de délivrance d'actif selon la revendication 4, **caractérisé en ce que** ledit polymère adhésif est un copolymère acrylique/acétate de vinyle.

6. Système de délivrance d'actif selon l'une quelconque des revendications 1 à 3, **caractérisé en ce que** ledit dérivé d'acide salicylique est solubilisé, au sein de ladite couche de polymère, dans un solvant et un gélifiant, ledit système comprenant en outre une couche adhésive perméable audit dérivé d'acide salicylique, et **en ce qu'**une membrane régulatrice est interposée entre ladite couche de polymère et ladite couche adhésive.

7. Utilisation du système de délivrance d'actif selon l'une quelconque des revendications 1 à 6pour la fabrication d'un produit destiné au traitement du psoriasis, de l'ichtyose, de l'eczéma, des kératoses actiniques, des dermatites séborrhéiques, de l'acné, des verrues ou des ulcères ou des hyperpigmentations de la peau.

8. Utilisation du système de délivrance d'actif selon l'une quelconque des revendications 1 à 6 pour la fabrication d'un produit destiné au traitement cosmétique des peaux grasses, des peaux jeunes à problèmes ou des points noirs et/ou pour assécher ou purifier la peau et/ou dans le traitement de l'acné.

9. Utilisation du système de délivrance d'actif selon l'une quelconque des revendications 1 à 6 pour la fabrication d'un produit à des fins de dépigmentation de la peau sans desquamation.

10. Procédé de traitement cosmétique consistant à appliquer sur la peau le système de délivrance d'actif selon l'une quelconque des revendications 1 à 6.

## Claims

1. System for the delivery of an active agent onto the skin, comprising, on a film structure, a composition based on a salicylic acid derivative satisfying the following formula (I) : 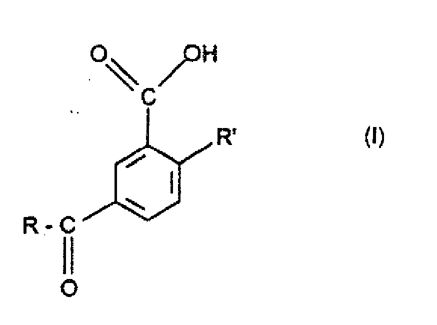 in which:
- R represents a linear, branched or cyclized saturated aliphatic chain having from 3 to 11 carbon atoms or an unsaturated chain having from 3 to 17 carbon atoms carrying one or more conjugated or non-conjugated double bonds, these various substituents possibly being substituted with one or more halogen atoms, with one or more trifluoromethyl groups in the free form or in the form esterified by an acid having from 1 to 6 carbon atoms, or else with a free carboxyl functional group or a carboxyl functional group esterified by a lower alcohol having from 1 to 6 carbon atoms, and
- R' represents a hydroxyl functional group or an ester functional group comprising a saturated or unsaturated chain having from 2 to 16 carbon atoms,
the said composition not containing a water-soluble active agent in the form of particles, **characterized in that** said film structure consists of, or includes, a natural or synthetic, woven or nonwoven fibrous material; or a sheet of plastic onto which is fastened a polymer layer containing said salicylic acid derivative.

2. Active-agent delivery system according to Claim 1, **characterized in that** R' is a hydroxyl functional group and R represents a saturated aliphatic chain having from 3 to 11 carbon atoms.

3. Active-agent delivery system according to Claim 2, **characterized in that** the said salicylic acid derivative is chosen from the group consisting of: 5-(n-hexanoyl)salicylic acid, 5-(n-octanoyl)salicylic acid, 5-(n-decanoyl)salicylic acid and 5-(n-dodecanoyl)salicylic acid.

4. Active-agent delivery system according to any of Claims 1 to 3, **characterized in that** the said polymer layer consists of an adhesive polymer.

5. Active-agent delivery system according to Claim 4, **characterized in that** the said adhesive polymer is an acrylic/vinyl acetate copolymer.

6. Active-agent delivery system according to any of Claims 1 to 3, **characterized in that** the said salicylic acid derivative is dissolved, within the said polymer layer, in a solvent and a gelling agent, the said system furthermore comprising an adhesive layer permeable to the said salicylic acid derivative, and **in that** a regulating membrane is interposed between the said polymer layer and the said adhesive layer.

7. Use of the active-agent delivery system according to any one of Claims 1 to 6 for the manufacture of a product intended for the treatment of psoriasis, ichthyosis, eczema, actinic keratoses, seborrhoeic dermatites, acne, warts or ulcers, or hyperpigmentations of the skin.

8. Use of the active-agent delivery system according to any one of Claims 1 to 6 for the preparation of a product intended for the cosmetic treatment of greasy skin, young skin with problems or blackheads and/or for drying or purifying the skin and/or in the treatment of acne.

9. Use of the active-agent delivery system according to any one of Claims 1 to 6 for the preparation of a product for the purposes of depigmentation of the skin without desquamation.

10. Method of cosmetic treatment consisting in applying the active-agent delivery system according to any one of Claims 1 to 6 to the skin.

## Patentansprüche

1. Abgabesystem für einen Wirkstoff auf die Haut, umfassend, auf einer Filmstruktur, eine Zusammensetzung auf der Grundlage eines Salicylsäurederivats, das der folgenden Formel (I) gehorcht: 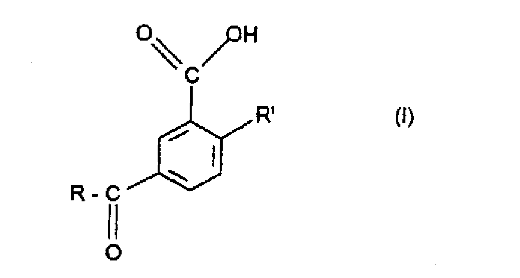 wobei
R eine lineare, verzweigte oder cyclische aliphatische gesättigte Kette mit 3 bis 11 Kohlenstoffatomen oder eine ungesättigte Kette mit 3 bis 17 Kohlenstoffatomen, die eine oder mehrere konjugierte oder nicht konjugierte Doppelbindungen trägt, darstellt, wobei diese verschiedenen Substituenten mit einem oder mehreren Halogenatomen, mit einer oder mehreren Trifluormethylgruppen in freier Form oder in mit einer Säure mit 1 bis 6 Kohlenstoffatomen veresterten Form oder auch mit einer Carboxylfunktion in freier Form oder in mit einem niederen Alkohol mit 1 bis 6 Kohlenstoffatomen veresterten Form substituiert sein können, und
R' eine Hydroxylfunktion oder eine Esterfunktion darstellt, die eine gesättigte oder ungesättigte Kette mit 2 bis 16 Kohlenstoffatomen umfasst,
wobei die Zusammensetzung keinen wasserlöslichen Wirkstoff in Form von Teilchen einschließt, **dadurch gekennzeichnet, dass** die Filmstruktur besteht aus oder umfasst ein faseriges, gewebtes oder nicht gewebtes, natürliches oder synthetisches Material; oder eine Kunststofffolie, auf der eine Polymerschicht fixiert ist, die das Salicylsäurederivat enthält.

2. Wirkstoff-Abgabesystem nach Anspruch 1, **dadurch gekennzeichnet, dass** R' eine Hydroxylfunktion ist und R eine aliphatische gesättigte Kette mit 3 bis 11 Kohlenstoffatomen darstellt.

3. Wirkstoff-Abgabesystem nach Anspruch 2, **dadurch gekennzeichnet, dass** das Salicylsäurederivat ausgewählt ist aus der Gruppe, die besteht aus: n-Hexanoyl-5-salicylsäure, n-Octanoyl-5-salicylsäure, n-Decanoyl-5-salicylsäure und n-Dodecanoyl-5-salicylsäure.

4. Wirkstoff-Abgabesystem nach einem der vorhergehenden Ansprüche 1 bis 3, **dadurch gekennzeichnet, dass** die Polymerschicht aus einem klebenden Polymer besteht.

5. Wirkstoff-Abgabesystem nach Anspruch 4, **dadurch gekennzeichnet, dass** das klebende Polymer ein Acryl/Vinylacetat-Copolymer ist.

6. Wirkstoff-Abgabesystem nach einem der Ansprüche 1 bis 3, **dadurch gekennzeichnet, dass** das Salicylsäurederivat in der Polymerschicht in einem Lösungsmittel und einem Gelbildner solubilisiert ist, wobei das System weiterhin eine Klebeschicht einschließt, die für das Salicylsäurederivat durchlässig ist, und **dadurch**, dass eine regulierende Membran zwischen der Polymerschicht und der Klebeschicht angeordnet ist.

7. Verwendung des Wirkstoff-Abgabesystems nach einem der Ansprüche 1 bis 6 zur Herstellung eines Produkts, das zur Behandlung von Psoriasis, Ichtiyose, Ekzem, aktinischen Keratosen, seborrhöischen Dermatiten, Akne, Warzen oder Ulzera oder Hyperpigmentierungen der Haut bestimmt ist.

8. Verwendung des Wirkstoff-Abgabesystems nach einem der Ansprüche 1 bis 6 zur Herstellung eines Produkts, das zur kosmetischen Behandlung von fettigen Häuten, jungen Problemhäuten oder Mitessern bestimmt ist, und/oder zum Austrocknen oder Reinigen der Haut und/oder bei der Behandlung der Akne.

9. Verwendung des Wirkstoff-Abgabesystems nach einem der Ansprüche 1 bis 6 zur Herstellung eines Produkts zu Depigmentierungszwecken der Haut ohne Desquamation.

10. Verfahren zur kosmetischen Behandlung, das darin besteht, das Wirkstoff-Abgabesystems nach einem der Ansprüche 1 bis 6 auf die Haut aufzutragen.
